# EUROPÄISCHE PATENTSCHRIFT

(11) **EP 2 736 555 B1**
(45) Veröffentlichungstag und Bekanntmachung des Hinweises auf die Patenterteilung: **05.07.2023**
(21) Anmeldenummer: 12745778.6
(22) Anmeldetag: 27.07.2012
(51) Int. Cl.: A61M 1/16, F04B 43/12

(54) **VERFAHREN SOWIE VORRICHTUNGEN ZUM ERFASSEN EINER DURCHLÄSSIGKEIT EINES IN EINE SCHLAUCHPUMPE EINGELEGTEN SCHLAUCHS**
METHOD AND DEVICES FOR DETECTING THE PERMABILITY OF A FLEXIBLE TUBE INSERTED INTO A PERISTALTIC PUMP
PROCÉDÉ ET DISPOSITIFS DE DÉTECTION DE LA PERMÉABILITÉ D'UN TUBE FLEXIBLE INSÉRÉ DANS UNE POMPE PÉRISTALTIQUE

(30) Priorität: 29.07.2011 DE 102011108778; 29.07.2011 US 201161512927 P
(43) Veröffentlichungstag der Anmeldung: 04.06.2014
(73) Patentinhaber: Fresenius Medical Care Deutschland GmbH, 61352 Bad Homburg (DE)
(72) Erfinder: GRONAU, Soeren, 64569 Nauheim (DE); MANKE, Joachim, 35792 Loehnberg (DE)
(74) Vertreter: Bobbert & Partner Patentanwälte PartmbB
(86) Internationale Anmeldenummer: PCT/EP2012/003195
(87) Internationale Veröffentlichungsnummer: WO 2013/017241

(56) Entgegenhaltungen:
- WO-A1-2010/020380
- DE-A1-102006 011 346
- US-A- 5 215 450
- US-A- 5 695 473
- US-A1- 2001 021 817

## Beschreibung

Die vorliegende Erfindung betrifft ein Verfahren gemäß Anspruch 1. Sie betrifft zudem eine Erfassungseinrichtung gemäß Anspruch 6 und eine medizinische Behandlungsvorrichtung gemäß Anspruch 9. Des Weiteren betrifft die vorliegende Erfindung ein digitales Speichermedium gemäß Anspruch 12, ein Computerprogramm-Produkt gemäß Anspruch 13 sowie ein Computerprogramm gemäß Anspruch 14.

Eine Aufgabe der vorliegenden Erfindung ist es, ein weiteres Verfahren zur Überprüfung der Funktion von Verdrängerkörpern einer Schlauchpumpe vorzuschlagen. Zudem sollen geeignete Vorrichtungen, ein geeignetes digitales Speichermedium, ein geeignetes Computerprogramm-Produkt und ein geeignetes Computerprogramm angegeben werden.

Die erfindungsgemäße Aufgabe wird durch ein Verfahren mit den Merkmalen des Anspruchs 1 gelöst. Sie wird ferner gelöst mittels der Erfassungseinrichtung mit den Merkmalen des Anspruchs 6, der Behandlungsvorrichtung mit den Merkmalen des Anspruchs 9, des digitalen Speichermediums mit den Merkmalen des Anspruchs 12, des Computerprogramm-Produkts mit den Merkmalen des Anspruchs 13 sowie des Computerprogramms mit den Merkmalen des Anspruchs 14.

Aus dem Stand der Technik sind verschiedene Verfahren bekannt, um die korrekte Funktion von Schlauchpumpen einer Blutbehandlungsvorrichtung zu überwachen. Beispielsweise kann die Drehzahl einer Schlauchpumpe mittels Durchflussratenwerten an einer Auslassleitung zum Patienten gesteuert werden, wie in der Anmeldung US 2001/0021817 A1 beschrieben.

Schlauchpumpen können auch mittels Verfahren überwacht werden, bei denen Messsignale aus den Verdrängerkörpern einer Schlauchpumpe untereinander verglichen werden und bei Abweichungen der einzelnen Messsignale oberhalb eines vorgegebenen Betrags, auf einen nicht ordnungsgemäßen Betrieb der Schlauchpumpe geschlossen wird, wie in der WO 2010/020380 A1 offenbart.

Bei anderen Schlauchpumpen des Stands der Technik wird die Funktion der Pumpe mittels deren Leistungsaufnahme oder mittels einer mit der Leistungsaufnahme korrelierenden physikalischen Größe überwacht. Aus dieser Größe werden ein Gleichanteil, der sich nicht periodisch ändert, und ein dem Gleichanteil überlagerter Wechselanteil bestimmt, der sich periodisch ändert. Auf der Grundlage des Gleichanteils und des Wechselanteils während des Betriebs der Pumpe wird ihre ordnungsgemäße Funktion überwacht, wie in der Anmeldung DE 10 2006 011 346 A1 beschrieben.

In der US 5,695,473 A ist ein Erkennungssystem für die Okklusion einer Infusionspumpe offenbart.

Ein Schlauchsystem mit Drucksensoren für peristaltische Pumpen ist aus der US 5,215,450 A bekannt.

Erfindungsgemäß wird somit in Anspruch 1 ein Verfahren zum Erfassen einer Durchlässigkeit eines Abschnitts eines in eine Schlauchpumpe eingelegten extrakorporalen Schlauchs vorgeschlagen. Das erfindungsgemäße Verfahren umfasst das Ineingriffbringen eines Verdrängerkörpers der Schlauchpumpe mit dem Abschnitt des Schlauchs derart, dass der Verdrängerkörper die Durchlässigkeit des Lumens des Abschnitts für ein Fluid und/oder die Querschnittsfläche des Lumens des Abschnitts vermindert. Das Verfahren umfasst ferner das Aufbauen, Einstellen oder Bewirken eines ersten Drucks oder das Bewirken einer ersten Druckveränderung innerhalb des Schlauchs auf einer ersten Seite des Abschnitts und/oder des in Eingriff stehenden Verdrängerkörpers. Weiter umfasst das erfindungsgemäße Verfahren jeweils das Messen eines zweiten Drucks oder einer zweiten Druckveränderung und/oder das Auswerten eines bereits gemessenen zweiten Drucks oder einer bereits gemessenen zweiten Druckveränderung auf einer zweiten Seite des Abschnitts und/oder des in Eingriff stehenden Verdrängerkörpers. Das Verfahren wird vor Aufnahme einer Behandlung eines Patienten durchgeführt und ggf. abgeschlossen.

Die erfindungsgemäße Erfassungseinrichtung ist konfiguriert zur Durchführung des erfindungsgemäßen Verfahrens und ist in Anspruch 6 definiert.

Die erfindungsgemäße medizinische oder medizintechnische Behandlungsvorrichtung (im Folgenden auch kurz: Behandlungsvorrichtung) ist in Anspruch 9 definiert und weist wenigstens eine erfindungsgemäße Erfassungseinrichtung auf und/oder ist hiermit in Signalübertragung verbunden oder steht mit dieser in einer Signalübertragungsbeziehung.

Ein erfindungsgemäßes digitales, insbesondere nichtflüchtiges, Speichermedium, insbesondere in Form eines maschinenlesbaren Trägers, insbesondere in Form einer Diskette, CD oder DVD oder EPROM, mit elektronisch oder optisch auslesbaren Steuersignalen ist in Anspruch 12 definiert und kann derart mit der erfindungsgemäßen Behandlungsvorrichtung zusammenwirken, dass die Schritte des erfindungsgemäßen Verfahrens veranlasst werden.

Dabei können alle, einige oder manche der durchgeführten Schritte des erfindungsgemäßen Verfahrens veranlasst werden.

Ein erfindungsgemäßes Computerprogramm-Produkt weist einen volatilen, flüchtigen oder auf einem maschinenlesbaren Träger gespeicherten Programmcode zur Veranlassung der Schritte des erfindungsgemäßen Verfahrens, wenn das Computerprogramm-Produkt auf der erfindungsgemäßen Behandlungsvorrichtungabläuft, auf und ist in Anspruch 13 definiert. Unter einem Computerprogramm-Produkt kann erfindungsgemäß beispielsweise ein auf einem Träger gespeichertes Computerprogramm, ein Embedded System als umfassendes System mit einem Computerprogramm (z. B. elektronisches Gerät mit einem Computerprogramm), ein Netzwerk von computerimplementierten Computerprogrammen (z. B. Client/Serversystem, Cloud Computing System, etc.), oder ein Computer, auf dem ein Computerprogramm geladen ist, abläuft, gespeichert ist, ausgeführt oder entwickelt wird, verstanden werden.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte und dergleichen sein.

Ein erfindungsgemäßes Computerprogramm ist in Anspruch 14 definiert und weist einen Programmcode auf zur Veranlassung der Schritte des erfindungsgemäßen Verfahrens, wenn das Computerprogramm auf der erfindungsgemäßen Behandlungsvorrichtung abläuft. Unter einem Computerprogramm kann erfindungsgemäß beispielsweise ein physikalisches, vertriebsfähiges Software-Produkt verstanden werden, welches ein Programm aufweist.

Auch für das erfindungsgemäße Computerprogramm-Produkt und das erfindungsgemäße Computerprogramm gilt, dass alle, einige oder manche der durchgeführten Schritte des erfindungsgemäßen Verfahrens veranlasst werden.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Vorteilhafte Weiterentwicklungen der vorliegenden Erfindung sind jeweils Gegenstand von Unteransprüchen und Ausführungsformen.

Erfindungsgemäße Ausführungsformen können eines oder mehrere der im Folgenden genannten Merkmale aufweisen.

In manchen erfindungsgemäßen Ausführungsformen dient das Verfahren zum Überwachen oder Überprüfen der Schlauchpumpe oder der Funktionsfähigkeit eines, mancher oder aller Verdrängerkörper der Schlauchpumpe.

In einigen Ausführungsformen dient das Verfahren dem Erfassen einer Durchlässigkeit des Lumens eines Abschnitts eines in die Schlauchpumpe eingelegten extrakorporalen Blutschlauchs.

In bestimmten Ausführungsformen dient das Verfahren dem Erfassen einer Durchlässigkeit des Lumens des Abschnitts des extrakorporalen Blutschlauchs, der in die Schlauchpumpe eingelegt ist.

In einigen Ausführungsformen dient das Verfahren dem Erfassen einer Durchlässigkeit des Lumes jenes Abschnitts des extrakorporalen Blutschlauchs, an dem der Verdrängerkörper oder die Verdrängungskörper zur Durchführung des erfindungsgemäßen Verfahrens mit dem Schlauch in Eingriff gebracht wird, werden oder wurde(n).

Die Schlauchpumpe ist in manchen Ausführungsformen eine peristaltische Pumpe, z. B. eine Rollenpumpe. Die Schlauchpumpe ist in bestimmten Ausführungsformen eine Blutpumpe.

In manchen Ausführungsformen wird das Verfahren bezogen auf einen konkreten Verdrängungskörper abschließend durchgeführt, während nur dieser Verdrängungskörper (nicht aber auch noch weitere Verdrängungskörper der Schlauchpumpe) das Lumen des Schlauchs komprimiert.

Die Schritte des Verfahrens werden in bestimmten Ausführungsformen in der hierin oder in den Ansprüchen jeweils angegebenen Reihenfolge durchgeführt.

In manchen Ausführungsformen sitzt der mindestens eine Verdrängerkörper auf einem Rotor und/oder ist im Gebrauch verschiebbar relativ zum Schlauch (in Längsrichtung oder tangential hierzu) bewegbar oder verschiebbar.

In bestimmten Ausführungsformen umfasst das Verfahren das Ineingriffbringen von genau einem oder nur einem Verdrängerkörper der Schlauchpumpe mit dem Abschnitt des Schlauchs, in anderen Ausführungsformen das gleichzeitige Ineingriffbringen mehrerer Verdrängerkörper.

"Ineingriffbringen" kann in manchen Ausführungsformen ein vollständiges oder teilweises Unterbinden der Durchgängigkeit des Lumens des Abschnitts in dessen Längserstreckung für ein Fluid, insbesondere für ein zu behandelndes medizinisches Fluid, oder für ein Messfluid wie Luft, Dialysat oder Substituat, bedeuten.

Ein Verdrängerkörper ist in einigen Ausführungsformen kein Ventil. In bestimmten erfindungsgemäßen Ausführungsformen ist der erste Druck ein definierter, bekannter oder vorbestimmter Druck.

In einigen Ausführungsformen ist der erste Druck ein Über-, in anderen Ausführungsformen ein Unterdruck.

In manchen Ausführungsformen ist eine erste Druckveränderung ein Druckanstieg, in anderen Ausführungsformen ein Druckabfall.

In manchen Ausführungsformen wird der Druckanstieg oder der Druckabfall unter Absperren oder Betätigen von Ventilen oder Drosseln, insbesondere zum Erzeugen von zwei gegenüber weiteren Schlauchabschnitten abgeschlossenen Schlauchsegmenten, bewirkt.

In manchen Ausführungsformen erfolgt das Überprüfen des Drucks, beispielsweise mittels Drucksensor, zwischen der Druckquelle, mit welcher der erste Druck oder die erste Druckveränderung bewirkt oder verändert wird, und dem Verdrängerkörper.

In gewissen Ausführungsformen wird oder wurde der Druck zwischen Verdrängerkörper und einer den Schlauch absperrenden (oder dessen Lumen vermindernden) Schlauchklemme, beispielsweise der arteriellen oder venösen Klemme, gemessen.

In bestimmten Ausführungsformen liegt die erste Seite - bezogen auf die Förderrichtung während des üblichen Betriebs der Schlauchpumpe - stromab des Abschnitts, welcher in Eingriff steht mit dem Verdrängerkörper. Die zweite Seite liegt in diesen Ausführungsformen stromauf dieses Abschnitts. In anderen Ausführungsformen liegt die erste Seite stromauf. Die zweite Seite liegt in diesen Ausführungsformen stromab dieses Abschnitts.

In einigen Ausführungsformen umfasst das Auswerten eines zweiten Drucks oder einer zweiten Druckveränderung über der Zeit eine entsprechende Messung, in anderen wird keine Messung durchgeführt, sondern auf bereits gemessene Werte oder anderweitig bekannte Werte zurückgegriffen. Dabei kann hierzu ein Druckwert eine direkte Angabe für einen Druck sein, beispielsweise in Form eines Messergebnisses. Unter einem Druckwert kann allerdings auch eine andere Größe als ein Druck zu verstehen sein, aus welchem auf den Druck geschlossen oder ein Druck berechnet werden kann.

In bestimmten Ausführungsformen erfolgt das Auswerten oder Überprüfen des zweiten Drucks oder der zweiten Druckveränderung beispielweise mit Hilfe von silikonbasierten piezoresistiven Sensoren, piezoelektrischen Sensoren, Membranmanometern, Biegebalkensensoren oder Widerstandskraftsensoren.

Die zweite Druckveränderung kann in manchen Ausführungsformen wie die erste Druckveränderung ein Druckabfall, in anderen ein Druckanstieg sein.

In manchen Ausführungsformen umfasst das Verfahren das Anhalten der Schlauchpumpe in der Position des Verdrängerkörpers, in welcher dieser in Eingriff mit dem Schlauch steht. In diesem Zustand, d. h. bei stehender, also nicht fördernder oder nicht rotierender Schlauchpumpe, kann in diesen Ausführungsformen der zweite Druck oder die zweite Druckveränderung gemessen werden.

In bestimmten Ausführungsformen umfasst das Verfahren das Bestimmen der Position des in Eingriff stehenden Verdrängerkörpers bezogen auf einen Abschnitt des Schlauchs oder einen Abschnitt des Pumprotorbetts oder des Stators der Schlauchpumpe. Ergänzend oder alternativ umfasst es das Anhalten der Schlauchpumpe derart, dass der Verdrängerkörper im gewünschten, beispielsweise teilweisen oder vollständigen oder maximalen Eingriff mit dem Schlauch steht.

Das Positionieren erfolgt in gewissen Ausführungsformen mittels eines oder mehrerer Magnete und/oder eines oder mehrerer Hall-Sensoren auf bekannte oder auf unten beschriebene Weise.

Das Verfahren umfasst in manchen Ausführungsformen das Vergleichen des zweiten Druckwerts, der zweiten Druckveränderung oder des zweiten Druckverlaufs mit entsprechenden, zuvor gespeicherten Werten oder Verläufen.

Das Verfahren umfasst in gewissen Ausführungsformen das Treffen einer Aussage darüber, ob ein Druckhaltetest bestanden ist oder nicht. Die Aussage kann getroffen werden anhand des Ergebnisses des Vergleichs mit zuvor gespeicherten Werten oder Verläufen.

In einigen Ausführungsformen wird das Verfahren mit jedem der im Betrieb der Schlauchpumpe in Eingriff mit dem Schlauch kommenden Verdrängerkörper durchgeführt. Dies erfolgt in manchen Ausführungsformen für die betreffenden Verdrängungskörper getrennt und unabhängig voneinander.

In manchen Ausführungsformen erfolgt das Verfahren nicht während der Behandlung des Patienten.

Die Erfassungseinrichtung ist in einigen Ausführungsformen konfiguriert, vorgesehen und/oder programmiert zum Erfassen einer Okklusionswirkung eines Verdrängerkörpers einer Schlauchpumpe auf einen Abschnitt eines in die Schlauchpumpe eingelegten Schlauchs.

In bestimmten Ausführungsformen ist die Erfassungseinrichtung eine Überwachungseinrichtung, eine Regel- oder Steuereinrichtung, ein Monitor oder eine Recheneinrichtung.

In gewissen Ausführungsformen weist die Erfassungseinrichtung Stelleinrichtungen, Messeinrichtungen, Steuereinrichtungen oder Regeleinrichtungen, Auswerteinrichtungen, Vergleichseinrichtungen, und/oder Speichereinrichtungen für Vergleichsdaten auf.

In manchen Ausführungsformen weist die Erfassungseinrichtung wenigstens eine Anzeigeeinrichtung zum Anzeigen eines Ergebnisses der Durchführung des erfindungsgemäßen Verfahrens auf.

In bestimmten Ausführungsformen weist die Erfassungseinrichtung wenigstens eine Alarmeinrichtung zum Ausgeben eines Alarms auf, welche vorgesehen und/oder konfiguriert ist zum Ausgeben eines Alarms für den Fall, dass der zweite Druck oder der zweite Druckverlauf nicht in einem vorbestimmten Bereich, Verlauf oder Wertebereich, oder jenseits von oberen und/oder unteren Grenzwerten liegt, oder vorgegebenen anderweitigen Bedingungen nicht genügt.

Ein Druckverlauf ist in einigen Ausführungsformen ein Verlauf des Drucks über der Zeit.

Die Behandlungsvorrichtung ist in manchen Ausführungsformen als Blutbehandlungsvorrichtung, insbesondere als Vorrichtung zur Apherese oder Dialyse, wiederum insbesondere zur Hämodialyse, Hämofiltration, Hämodiafiltration, oder zur Akutdialyse ausgestaltet.

In einigen Ausführungsformen der Behandlungsvorrichtung ist wenigstens einer der Verdrängerkörper als Rolle und die peristaltische Schlauchpumpe als Rollenpumpe ausgestaltet.

In manchen Ausführungsformen wird das Verfahren bei stehender Pumpe durchgeführt.

Einige oder alle Ausführungsformen können einen oder mehrere der oben oder im Folgenden genannten Vorteile aufweisen.

So kann, da das erfindungsgemäße Verfahren beim Vorbereiten oder Aufrüsten der Behandlungsvorrichtung erfolgen kann, ein technischer Fehler bereits erkannt werden, bevor der Patient konnektiert ist und bevor Blut in Kontakt mit der Vorrichtung sowie dem extrakorporalen Blutkreislauf gelangt ist. Letzteres vermeidet einen unnötigen Mehrverbrauch an Einwegartikeln. Es erlaubt ferner ein einfaches, da frühzeitiges Austauschen von defekten Komponenten im Fehlerfall.

Aufgrund der Messanordnung kann mittels eines kleinen Messvolumens oder eines kurzen Abschnitts des Schlauchs, welcher zur ersten Seite hin mittels des zu testenden Verdrängerkörpers okkludiert wird, sehr genau gemessen werden.

Neben anderen Einsatzgebieten werden Schlauchpumpen u. a. in der extrakorporalen Blutbehandlung wie der Dialyse zur Förderung eines Fluids, hier Blut, eingesetzt. Stand der Technik sind federgelagerte Rollen, welche den Maximaldruck der Pumpe - welcher zudem vom Pumpschlauchdurchmesser abhängt - begrenzen sollen. Mit wachsender mechanischer Komplexität des Pumprotors wächst üblicherweise die Anzahl möglicher Fehlerquellen, so auch bei Schlauchpumpen. Daher ist es sinnvoll, die Funktionstüchtigkeit des Rotors zu prüfen. In der WO 2010/020380 A1 ist ein Verfahren offenbart, um den Pumprotor im laufenden Pumpenbetrieb zu überwachen. Dabei werden Druckverläufe, hervorgerufen durch die im Moment der Betrachtung sich im Eingriff befindenden Rolle erhoben und mit Druckverläufen anderer Rollen verglichen. Treten Abweichungen auf, wird auf einen Defekt des Pumprotors geschlossen. Ein sich gleichermaßen auf beide (im Sinne von allen) Rollen auswirkender Schaden kann prinzipbedingt nicht erkannt werden. Dies ist mittels bestimmter Ausführungsformen des erfindungsgemäßen Verfahrens anders. In solchen Ausführungsformen können vorteilhaft auch Defekte oder Fehler erkannt werden, welche zur Schlechtleistung aller beteiligten oder auf ihre Funktion überprüften Verdrängerkörper führen. Ein solcher Fehler kann im Verwenden eines - beispielsweise aufgrund seines Schlauchdurchmessers oder Materials - für die konkrete Anwendung nicht geeigneten Schlauchs bestehen. Ein solcher Fehler kann ferner z. B. durch Riefen in der Pumpschlauch-Innenschicht bestehen, welche durch einen Produktionsfehler verursacht sein können. Aufgrund der erfindungsgemäß möglichen Absolutmessungen, welche für alle Verdrängerkörper jeweils unabhängig voneinander durchgeführt werden können, kann somit auch ein sich auf alle Verdrängerkörper gleichartig auswirkender Schaden vorteilhaft erkannt werden.

Auf diese Weise kann neben einer Überprüfung der Funktionsfähigkeit der Verdrängerkörper auch eine Funktionsüberprüfung oder Qualitätskontrolle des Pumpschlauchs durchgeführt werden. Hierzu sind vorteilhafterweise kein eigener Test und keine eigenen Verfahrensschritte durchzuführen.

Zudem ist es auch möglich, eine Funktionsbeeinträchtigung nur eines Verdrängerkörpers zu erkennen. Neben der zuvor beschriebenen Absolutmessung ist daher auch eine Relativmessung möglich. So kann beispielsweise bei ausfahrbaren Rollen unter Einsatz bestimmter Ausführungsformen der vorliegenden Erfindung nachgewiesen werden, dass alle überprüften Rollen vollständig oder bestimmungsgemäß ausgefahren werden können. Dies lässt sich mittels des jeweils gemessenen Okklusionsdrucks - beispielsweise bei Pumpenstillstand - nachweisen, wenn dieser zuvor bestimmten Kriterien genügt, z. B. also oberhalb des zulässigen zweiten Drucks oder des Prüfdrucks oder dessen Verlaufs liegt. Mechanische Defekte am Pumpenrotor wie verklemmte Schwingen oder ein Federbruch, welche nur einzelne Rollen, Finger oder Stifte betreffen, können auf diese Weise detektiert werden. Ferner lässt sich ein Produktionsfehler erkennen, der z. B. in der Verwendung von zu schwachen oder anderweitig ungeeigneten Federn bei einer oder allen Rollen besteht.

Eine nicht oder nicht vollständig okkludierende Blutpumpe kann neben einer Förderratenabweichung und einer ebenfalls unerwünschten Rückwärtsförderung aus dem mittels des Verdrängerkörpers komprimierten Abschnitt heraus unter Umständen zu einer Schädigung des extrakorporal geförderten Bluts führen. Auch solche Fehler oder Gefahren können mittels des erfindungsgemäßen Verfahrens frühzeitig erkannt werden.

Die vorliegende Erfindung wird im Folgenden anhand der beigefügten Zeichnungen, in welcher identische Bezugszeichen gleiche oder ähnliche Bauteile bezeichnen, exemplarisch erläutert. In den zum Teil stark vereinfachten Figuren gilt:
- Fig. 1: zeigt schematisch vereinfacht einen extrakorporalen Blutkreislauf mit einer Schlauchpumpe, deren Okklusionswirkung mittels des erfindungsgemäßen Verfahrens geprüft werden kann;
- Fig. 2: zeigt einen Pumprotor, welcher Teil einer Schlauchpumpe einer erfindungsgemäßen Behandlungsvorrichtung sein kann, in perspektivischer Ansicht, auf seiner Rückseite, der Ankoppelfläche, liegend;
- Fig. 3: zeigt den Pumprotor der Fig. 2 mit seitlichem Blick auf dessen Ankoppelfläche; und
- Fig. 4: zeigt Druck- und Volumenstromverhältnisse oder Druck- und Volumenstromänderungen bei Durchführung des erfindungsgemäßen Verfahrens.

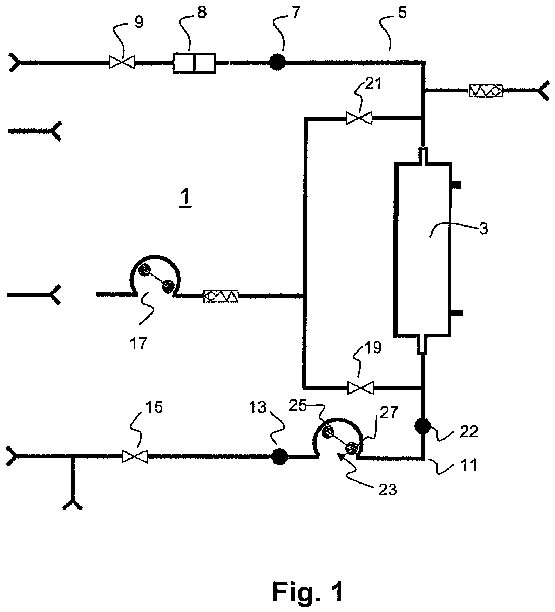

**Fig. 1** zeigt schematisch vereinfacht einen extrakorporalen Blutkreislauf 1, welcher Teil einer Blutkassette ist. Der Blutkreislauf 1 ist wenigstens mit einem Dialysator 3, einem in einer venösen Blutleitung 5 angeordneten venösen Druckmesser 7, einem optischen Detektor oder Luftblasendetektor 8, einer venösen Klemme 9, einem in einer arteriellen Blutleitung 11 angeordneten arteriellen Druckmesser 13, einer arteriellen Klemme 15, einer Substituatpumpe 17, einem Prädilutionsventil 19 und einem Postdilutionsventil 21 ausgestaltet oder hiermit verbunden. Die arterielle Blutleitung 11 weist eine Messstelle 22 auf, welche geeignet oder vorgesehen ist zum Messen eines Präfilterdrucks oder eines Drucks, welcher vor dem oder stromaufwärts des Dialysator 3 herrscht. Weitere Einrichtungen wie vor allem Ports und Konnektoren - sowie deren durch die Form der Blutkassette bedingte Lage - sind in Fig. 1 erkennbar, sie sind jedoch nicht erfindungsrelevant.

Erfindungsrelevant ist hingegen die mit dem Bezugszeichen 23 bezeichnete Schlauchpumpe zum Fördern von Blut, welche ebenso wie die Substituatpumpe 17 Teil einer erfindungsgemäßen Behandlungsvorrichtung ist und welche mittels des beanspruchten Verfahrens überprüft werden kann. Die Schlauchpumpe 23 ist exemplarisch als Rollenpumpe ausgeführt. Sie weist im Beispiel der Fig. 1 erkennbar zwei Rollen 25 und 27 auf, als Beispiele für Verdrängerkörper. Für den Fachmann ist bereits aus Fig. 1 erkennbar, dass die Anzahl der Verdrängerkörper oder Rollen 25, 27 erfindungsgemäß nicht auf zwei beschränkt ist. Auch drei oder mehr Verdrängerkörper oder Rollen können erfindungsgemäß vorgesehen sein und überprüft werden. Für den Fachmann ist ferner erkennbar, dass die Erfindung nicht auf ein Überprüfen einer in einer Kreisbewegung arbeitenden Schlauchpumpe beschränkt ist. Tatsächlich ist das erfindungsgemäße Verfahren beispielsweise auch zur Verwendung an einer Schlauchpumpe vorgesehen, welche mittels Finger, nicht mittels Rollen, fördert. Ein Beispiel solcher Pumpen ist in der US 6,558,347 B1 offenbart.

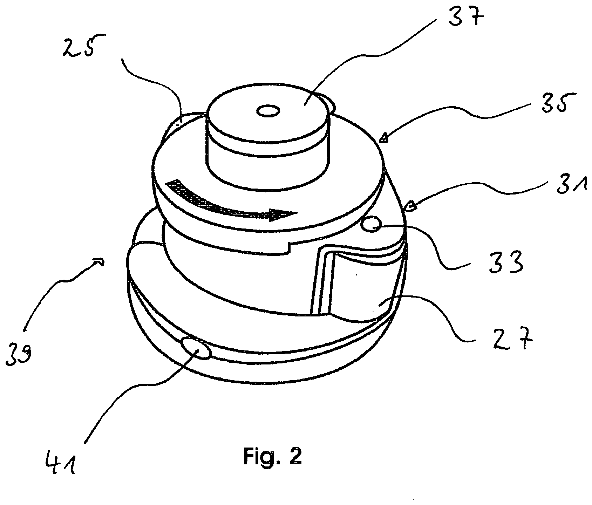

**Fig. 2** zeigt einen Pumprotor 31, welcher Teil einer Schlauchpumpe einer erfindungsgemäßen Blutbehandlungsvorrichtung sein kann, in perspektivischer Ansicht von vorne oben. Der Pumprotor 31 weist beispielhaft zwei jeweils mittels einer Achse 33 gelagerte Rollen 25 und 27 auf. Es könnten allerdings auch mehr als zwei Rollen vorgesehen sein. Die Rollen könnten anderer Gestalt sein. Sie müssten nicht mittels Achsen gelagert sein.

Das im Gebrauch des Pumprotors 31 der Behandlungsvorrichtung abgewandte Oberteil 35 ist mit einem Drehknopf 37 zur manuellen Drehbarkeit des Pumprotors 31 ausgestaltet. Der Pumprotor 31 kann mittels des Drehknopfs 37 beispielsweise während des Einlegens des in Fig. 2 nicht dargestellten Blutschlauchs in die Schlauchpumpe von Hand gedreht werden.

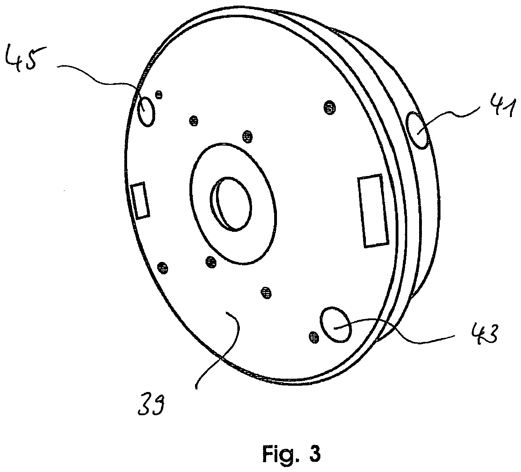

An seinem Unterteil 39, dem im Betrieb des Pumprotors 31 der Behandlungsvorrichtung zugewandten Teil des Pumprotors 31, weist der Pumprotor 31 drei Magneten an unterschiedlichen Magnetpositionen auf. Es könnten allerdings auch mehr als drei Magnetpositionen vorgesehen sein, insbesondere kann die Anzahl der Magnetpositionen mit der Anzahl der vorgesehenen Rollen korrelieren. Eine der hier exemplarisch vorgesehenen Magnetpositionen, die Magnetposition 41, ist in Fig. 2 zu erkennen. Sie ist in eine Stirnseite des Unterteils 39 eingearbeitet. Die übrigen beiden Magnetpositionen 43 und 45 liegen auf oder in der unteren Stirnfläche des Pumprotors und damit auf oder in einer Ankoppelfläche, mittels welcher der Pumprotor 31 funktionell mit der Behandlungsvorrichtung verbunden ("angekoppelt") wird. Die übrigen beiden Magnetpositionen 43 und 45 sind daher in Fig. 2 nicht erkennbar. Sie sind jedoch in Fig. 3 erkennbar, welche den Pumprotor 31 der Fig. 2 mit Blick von hinten seitlich, also auf die Ankoppelfläche, zeigt.

Das in den Figuren nicht gezeigte Pumprotorbett weist an entsprechenden Stellen insgesamt zwei Hall-Sensoren auf. Ein erster Hall-Sensor liegt an einer Stirnseite des Pumprotorbetts, ein zweiter Hall-Sensor liegt an der Ankoppelfläche des Unterteils 39 des Pumprotors 31, dem Rotorboden.

Wird die einzelne, seitlich am Pumprotor 31 vorgesehene Magnetposition 41 am ersten Hall-Sensor vorbei bewegt, so lässt sich die absolute Position und damit die sich zu diesem Zeitpunkt aktuell im Eingriff mit dem Schlauch befindende Rolle erfassen.

Zur Feinauflösung der Rotorposition erzeugt der Pumpenmotor in bestimmten erfindungsgemäßen Ausführungsformen ein Tachosignal. Mit dessen Hilfe kann der seit dem letzten Hall-Durchgang überstrichene Winkel ermittelt werden, sofern die Zahl der Tachopulse je Winkelgrad bekannt ist.

Mithilfe dieser Sensorik kann die Position des Pumprotors 31 mit ausreichender Genauigkeit ermittelt werden. Ebenso kann hierauf basierend der Pumprotor 31 beliebig positioniert werden.

Alternativ können für die Positionierung der ersten Rolle 25 einer der an der Ankoppelfläche des Unterteils 39 des Pumprotors 31, dem Rotorboden, befindlichen Magnete der Magnetpositionen 43 und 45 am zweiten Hall-Sensor, welcher in einer Ankoppelfläche des Pumprotorbetts liegt, vorbei bewegt werden. Anschließend kann der Pumprotor 31 zur Überprüfung der zweiten Rolle 27 mittels des Tachosignals um 180° um seine Drehachse weitergedreht werden.

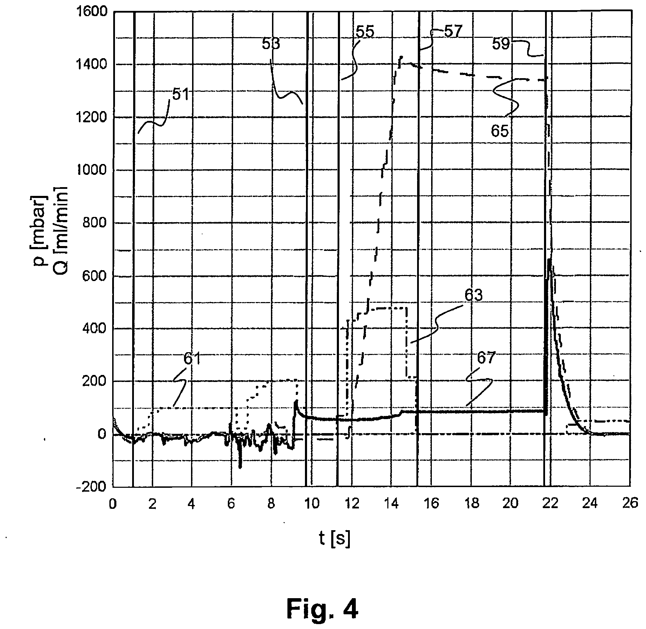

**Fig. 4** zeigt Druck- und Volumenstromverhältnisse oder Druck- und Volumenstromänderungen sowie deren Verläufe bei einer Rollenmessung oder Rollenüberprüfung mittels des beanspruchten Verfahrens. Dabei sind ein Blutpumpenfluss 61 und ein Substituatpumpenfluss 63 jeweils in ml/min angegeben. Ein Druck 65 vor der Pumpe und ein Druck 67 hinter der Pumpe sind jeweils in mbar angegeben. Nachdem der extrakorporale Blutkreislauf 1 der Fig. 1 gefüllt und entlüftet worden ist, wird - noch bevor der Patient angeschlossen wird - dessen Dichtigkeit geprüft. Parallel wird das beanspruchte Verfahren, welches in der hier beschriebenen Ausführungsform auch als Rotorstillstandsleckageprüfung bezeichnet werden kann, da die überprüfte Schlauchpumpe dabei steht, durchgeführt.

Zur Durchführung der hier beschriebenen Okklusionsprüfung wird zunächst eine der beiden Rollen, z. B. die erste Rolle 25, in Eingriff mit dem Schlauch gebracht. Dies kann nach Positionierung mit Hilfe eines oder beider der oben genannten Hall-Sensoren zum Erfassen der Winkelposition des Pumprotors 31 erfolgen, wie oben beschrieben ist.

In der Darstellung der Fig. 4 beginnt die Positionierung der ersten Rolle 25, während welcher diese in Eingriff mit dem Schlauch gebracht wird, zu dem mit dem Bezugszeichen 51 markierten Zeitpunkt und endet an dem mit dem Bezugszeichen 53 markierten Zeitpunkt. Nach Positionierung steht nur eine Rolle, hier die Rolle 25, in Eingriff mit dem in den Figuren nicht dargestellten Schlauch.

In diesem Zustand werden die arterielle Klemme 15 und die venöse Klemme 9 geschlossen. Dialysatzulaufventile und Dialysatablaufventile vom und zum Dialysator werden ebenfalls geschlossen. Somit entstehen ein erstes und ein zweites Schlauchsegment, welche beide nicht in Fluidkommunikation mit anderen Schlauchabschnitten stehen oder diesen gegenüber abgesperrt sind.

Mittels der jetzt fördernden Substituatpumpe 17 kann nun ein erster Druck im ersten Schlauchsegment des extrakorporalen Blutkreislaufs 1 zwischen Schlauchpumpe 23 und venöser Klemme 9 aufgebaut werden. Das zwischen Rolle 25 der Pumpe und arterieller Klemme 15 eingeschlossene Volumenkompartiment des zweiten Schlauchsegments ist klein und hat eine geringe Compliance (hohe Steifigkeit), wodurch bereits kleine Flüssigkeitsübertritte in das zweite Schlauchsegment hinein hohe Druckänderungen dort hervorrufen. Im zweiten Schlauchsegment wird ein zweiter Druck gemessen.

Der Beginn des Druckaufbaus im ersten Schlauchsegment, also auf der ersten Seite, beginnt an dem mit dem Bezugszeichen 55 markierten Zeitpunkt. Gleichzeitig oder kurz danach beginnt die Bewertung des Druckanstiegs im zweiten Schlauchsegment, also auf der zweiten Seite. An dem mit dem Bezugszeichen 57 bezeichneten Zeitpunkt oder kurz zuvor endet der Druckaufbau. Zum Zeitpunkt 59 beginnt der Druckabbau, die Bewertung des Druckanstiegs endet dann.

Während der Druckaufbau- und -haltezeit wird der Druck zwischen arterieller Klemme 15 und Rolle 25, gemessen mit dem arteriellen Druckmesser 13, ausgewertet. Übersteigt dessen Änderung einen definierten Grenzwert (z. B. 200 mmHg) so schlägt der Test fehl. Eine entsprechende Anzeige und/oder Warnung oder ein entsprechender Alarm (optisch, akustisch, Systemteilabschaltung, usw.) kann erfolgen. Ein Defekt des Pumprotors 31 oder ein Problem mit dem Schlauch kann nicht ausgeschlossen werden.

Wird die Prüfung bestanden, - übersteigt der Druck während der Testzeit den zulässigen Wert nicht - ist die Prüfung der ersten Rolle 25 abgeschlossen. Der Druck im extrakorporalen Blutkreislauf 1 wird durch Öffnen der venösen Klemme 9 abgebaut und die Prüfung der zweiten Rolle 27 wird vorbereitet.

Zur Prüfung der zweiten Rolle 27 genügt es, den Pumprotor 31 um 180° zu drehen, wodurch aufgrund der Symmetrie des Pumprotors 31 die zweite Rolle 27 exakt positioniert wird. Es besteht natürlich auch die Möglichkeit mithilfe der Hall-Sensoren eine absolute Positionierung der zweiten Rolle 27 durchzuführen.

Nach der Positionierung der zweiten Rolle 27 wird der zur Prüfung der ersten Rolle 25 skizzierte Ablauf erneut durchlaufen. Wurde der Test, also das Verfahren, auch für die zweite Rolle 27 bestanden, so kann mit weiteren Tests oder mit dem Vorbereiten der Vorrichtung für den Anschluss des Patienten fortgefahren werden.

### Verwendete Bezugszeichen:

- 1: extrakorporaler Blutkreislauf
- 3: Dialysator
- 5: venöse Blutleitung
- 7: venöser Druckmesser
- 8: Luftblasendetektor
- 9: venöse Klemme
- 11: arterielle Blutleitung
- 13: arterieller Druckmesser
- 15: arterielle Klemme
- 17: Substituatpumpe
- 19: Prädilutionsventil
- 21: Postdilutionsventil
- 22: Messstelle zum Messen eines Präfilterdrucks
- 23: Schlauchpumpe
- 25, 27: Rollen/Verdrängerkörper
- 31: Pumprotor
- 33: Achse
- 35: Oberteil
- 37: Drehknopf
- 39: Unterteil
- 41, 43, 45: Magnetpositionen
- 51: Beginn der Positionierung der ersten Rolle
- 53: Ende der Positionierung der ersten Rolle
- 55: Beginn des Druckaufbaus und Beginn der Bewertung eines Druckanstiegs
- 57: Ende des Druckaufbaus
- 59: Ende der Bewertung eines Druckanstiegs und Ende der Bewertung des Druckanstiegs

## Patentansprüche

1. Verfahren zum Erfassen einer Durchlässigkeit eines Abschnitts eines in eine Schlauchpumpe (23) eingelegten extrakorporalen Schlauchs, mit den Schritten:
- Ineingriffbringen eines Verdrängerkörpers (25, 27) der Schlauchpumpe (23) mit einem Abschnitt des Schlauchs derart, dass der Verdrängerkörper (25, 27) die Durchlässigkeit des Abschnitts vermindert;
- Bewirken oder Verändern eines ersten Drucks oder einer ersten Druckveränderung innerhalb des Schlauchs auf einer ersten Seite des Abschnitts und/oder auf einer ersten Seite des in Eingriff stehenden Verdrängerkörpers (25, 27), wobei der erste Druck oder die erste Druckveränderung durch eine Substituatpumpe und/oder das Unterbrechen von Fluidverbindungen bewirkt wird;
- Auswerten eines zweiten Drucks oder einer zweiten Druckveränderung, welcher oder welche auf einer zweiten Seite des Abschnitts und/oder auf einer zweiten Seite des in Eingriff stehenden Verdrängerkörpers (25, 27) herrscht oder messbar ist,
wobei das Verfahren vor Aufnahme einer Behandlung eines Patienten durchgeführt wird.

2. Verfahren nach Anspruch 1, mit dem weiteren Schritt:
- Anhalten der Schlauchpumpe (23) in der Position des Verdrängerkörpers (25, 27), in welcher dieser in Eingriff mit dem Schlauch steht, zum Messen des zweiten Drucks oder der zweiten Druckveränderung.

3. Verfahren nach Anspruch 1 oder 2, mit den weiteren Schritten:
- Bestimmen der Position des in Eingriff stehenden Verdrängerkörpers (25, 27) mittels Messsignalen, und/oder
- Anhalten der Schlauchpumpe (23) in Abhängigkeit der Messsignale bezüglich der Position des Verdrängerkörpers (25, 27) derart, dass der Verdrängerkörper (25, 27) in Eingriff mit dem Schlauch steht und/oder in Eingriff stehen bleibt.

4. Verfahren nach einem der Ansprüche 1 bis 3, mit dem Schritt:
- Vergleichen des zweiten Druckwerts oder der zweiten Druckveränderung mit zuvor gespeicherten Werten, Schwellenwerten, Bereichen oder Verläufen.

5. Verfahren nach einem der Ansprüche 1 bis 4, mit dem Schritt:
- Durchführen des Verfahrens mit jedem der im Betrieb der Schlauchpumpe (23) in Eingriff mit dem Schlauch kommenden Verdrängerkörper (25, 27).

6. Erfassungseinrichtung konfiguriert zum Durchführen eines Verfahrens nach einem der vorangegangenen Ansprüche, wobei die Erfassungsrichtung umfasst:
- eine Einrichtung zum Ineingriffbringen eines Verdrängerkörpers (25, 27) einer Schlauchpumpe (23) mit einem Abschnitt eines Schlauchs;
- eine Einrichtung zum Bewirken oder Verändern eines ersten Drucks oder einer ersten Druckveränderung innerhalb des Schlauchs auf einer ersten Seite des Abschnitts und/oder auf einer ersten Seite des in Eingriff stehenden Verdrängerkörpers (25, 27); und
- eine Einrichtung zum Auswerten eines zweiten Drucks oder einer zweiten Druckveränderung, welcher oder welche auf einer zweiten Seite des Abschnitts und/oder auf einer zweiten Seite des in Eingriff stehenden Verdrängerkörpers (25, 27) herrscht oder messbar ist.

7. Erfassungseinrichtung nach Anspruch 6, aufweisend wenigstens eine Anzeigeeinrichtung zum Anzeigen eines Ergebnisses der Durchführung des Verfahrens nach einem der Ansprüche 1 bis 5.

8. Erfassungseinrichtung nach Anspruch 6 oder 7, aufweisend wenigstens eine Alarmeinrichtung zum Ausgeben eines Alarms, wobei die Alarmeinrichtung vorgesehen ist zum Ausgeben eines Alarms für den Fall, dass das Ergebnis der Durchführung des Verfahrens nach einem der Ansprüche 1 bis 5 nicht in einem vorbestimmten Wertebereich oder Bereich liegt.

9. Medizinische Behandlungsvorrichtung, welche wenigstens eine Erfassungseinrichtung nach Anspruch 6 aufweist und/oder mit dieser in Signalübertragung steht oder zur Signalübertragung verbunden ist.

10. Medizinische Behandlungsvorrichtung nach Anspruch 9, welche als Blutbehandlungsvorrichtung, insbesondere als Vorrichtung zur Apherese oder Dialyse, wiederum insbesondere zur Hämodialyse, Hämofiltration, Hämodiafiltration, ausgestaltet ist.

11. Medizinische Behandlungsvorrichtung nach einem der Ansprüche 9 und 10, wobei wenigstens einer der Verdrängerkörper (25, 27) als Rolle ausgestaltet ist, und wobei die Schlauchpumpe (23) als Rollenpumpe ausgestaltet ist.

12. Digitales Speichermedium, insbesondere in Form einer Diskette, CD oder DVD oder EPROM, mit elektronisch auslesbaren Steuersignalen, konfiguriert, um derart mit der Behandlungsvorrichtung nach Anspruch 9 zusammenzuwirken, dass die Schritte eines erfindungsgemäßen Verfahrens nach einem der Ansprüche 1 bis 5 veranlasst werden.

13. Computerprogramm-Produkt mit einem auf einem maschinenlesbaren Träger gespeicherten Programmcode zur Veranlassung der Schritte des erfindungsgemäßen Verfahrens gemäß einem der Ansprüche 1 bis 5, wenn das Computerprogramm-Produkt auf der Behandlungsvorrichtung nach Anspruch 9 abläuft.

14. Computerprogramm mit einem Programmcode zur Veranlassung der maschinellen Schritte eines erfindungsgemäßen Verfahrens gemäß einem der Ansprüche 1 bis 5, wenn das Computerprogramm auf der Behandlungsvorrichtung nach Anspruch 9 abläuft.

## Claims

1. A method for detecting a permeability of a section of an extracorporeal tube inserted in a tube pump (23), comprising the steps of:
- meshing a displacer (25, 27) of the tube pump (23) with a section of the tube such that the displacer (25, 27) reduces the permeability of the section;
- effecting or changing a first pressure or a first pressure change within the tube on a first side of the section and/or on a first side of the meshed displacer (25, 27), the first pressure or the first pressure change being effected by a substituate pump and/or by the interruption of fluid connections;
- evaluating a second pressure or a second pressure change, which prevails or is measurable on a second side of the section and/or on a second side of the meshed displacer (25, 27),
wherein the method is carried out before starting a treatment of a patient.

2. The method according to claim 1, comprising the further step of:
- stopping the tube pump (23) in the position in which the displacer (25, 27) is meshed with the tube for measuring the second pressure or the second pressure change.

3. The method according to claim 1 or 2, comprising the further steps of:
- determining the position of the meshed displacer (25, 27) by means of measurement signals, and/or
- stopping the tube pump (23) depending on the measurement signals with regard to the position of the displacer (25, 27) such that the displacer (25, 27) is and/or remains meshed with the tube.

4. The method according to anyone of claims 1 to 3, comprising the step of:
- comparing the second pressure value or the second pressure change with previously stored values, threshold values, ranges or gradients.

5. The method according to anyone of claims 1 to 4, comprising the step of:
- executing the method with each of the displacers (25, 27) coming into mesh with the tube during the operation of the tube pump (23).

6. A detection device configured to execute a method according to anyone of the preceding claims, wherein the detection device comprises:
- a device for meshing a displacer (25, 27) of a tube pump (23) with a section of a tube;
- a device for effecting or changing a first pressure or a first pressure change within the tube on a first side of the section and/or on a first side of the meshed displacer (25, 27); and
- a device for evaluating a second pressure or a second pressure change, which prevails or is measurable on a second side of the section and/or on a second side of the meshed displacer (25, 27).

7. The detection device according to claim 6, comprising at least one display device for displaying a result of the execution of the method according to anyone of claims 1 to 5.

8. The detection device according to claim 6 or 7, comprising at least one alarm device for outputting an alarm,
the alarm device being provided for outputting an alarm in the event that the result of the execution of the method according to anyone of claims 1 to 5 does not lie in a predetermined values range or range.

9. A medical treatment apparatus which comprises at least one detection device according to claim 6 and/or which is in signal transmission or which is connected therewith for signal transmission.

10. The medical treatment apparatus according to claim 9, which is designed as a blood treatment apparatus, in particular as an apparatus for apheresis or dialysis, again in particular for hemodialysis, hemofiltration, hemodiafiltration.

11. The medical treatment apparatus according to anyone of claims 9 and 10, wherein at least one of the displacers (25, 27) is designed as a roller, and wherein the tube pump (23) is designed as a roller pump.

12. A digital storage medium, in particular in the form of a disk, CD or DVD or EPROM, with electronically readable control signals, configured to interact with the treatment apparatus according to claim 9 so as to prompt the steps of a method based on the invention according to anyone of claims 1 to 5.

13. A computer program product comprising a program code stored on a machine-readable carrier for prompting the steps of the method based on the invention according to anyone of claims 1 to 5, when the computer program product is running on the treatment apparatus according to claim 9.

14. A computer program comprising a program code for prompting the automatic steps of a method based on the invention according to anyone of claims 1 to 5, when the computer program is running on the treatment apparatus according to claim 9.

## Revendications

1. Un procédé de détection d'une perméabilité d'une section d'un tube extracorporel inséré dans une pompe tubulaire (23), comprenant les étapes suivantes:
- l'engagement d'un corps de déplacement (25, 27) de la pompe tubulaire (23) avec une section du tube de manière à ce que le corps de déplacement (25, 27) réduise la perméabilité de la section;
- l'application ou la modification d'une première pression ou d'une première variation de pression à l'intérieur du tube sur un premier côté de la section et/ou sur un premier côté du corps de déplacement (25, 27) engagé, la première pression ou la première variation de pression étant appliquée par une pompe à substitut et/ou par l'interruption des communications fluidiques;
- l'évaluation d'une seconde pression ou d'une seconde variation de pression qui règne ou qui est mesurable sur un second côté de la section et/ou sur un second côté du corps de déplacement (25, 27) engagé,
où le procédé est exécuté avant de commencer un traitement d'un patient.

2. Le procédé selon la première revendication, comprenant en outre l'étape consistant à:
- l'arrêt de la pompe tubulaire (23) dans la position dans laquelle le corps de déplacement (25, 27) est engagé avec le tube, afin de mesurer la seconde pression ou la seconde variation de pression.

3. Le procédé selon la revendication 1 ou 2, comprenant en outre l'étape consistant à:
- la détermination de la position du corps de déplacement (25, 27) engagé, au moyen de signaux de mesure, et/ou
- l'arrêt de la pompe tubulaire (23) en fonction des signaux de mesure par rapport à la position du corps de déplacement (25, 27), de manière à ce que le corps de déplacement (25, 27) soit et/ou reste engagé avec le tube.

4. Le procédé selon l'une quelconque des revendications 1 à 3, comprenant l'étape suivante:
- la comparaison de la seconde valeur de pression ou de la seconde variation de pression avec des valeurs, des valeurs seuils, des plages ou des gradients préalablement enregistrés.

5. Le procédé selon l'une quelconque des revendications 1 à 4, comprenant l'étape suivante:
- l'exécution du procédé avec chacun des corps de déplacement (25, 27) s'engageant avec le tube lors du fonctionnement de la pompe tubulaire (23).

6. Un dispositif de détection configuré pour exécuter un procédé selon l'une quelconque des revendications précédentes, où le dispositif de détection comprend:
- un dispositif pour engager un corps de déplacement (25, 27) d'une pompe tubulaire (23) avec une section d'un tube;
- un dispositif pour appliquer ou modifier une première pression ou une première variation de pression à l'intérieur du tube sur un premier côté de la section et/ou sur un premier côté du corps de déplacement (25, 27) engagé; et
- un dispositif pour évaluer une seconde pression ou une seconde variation de pression qui règne ou qui est mesurable sur un second côté de la section et/ou sur un second côté du corps de déplacement (25, 27) engagé.

7. Le dispositif de détection selon la revendication 6, comprenant au moins un dispositif d'affichage pour afficher un résultat de l'exécution du procédé selon l'une quelconque des revendications 1 à 5.

8. Le dispositif de détection selon la revendication 6 ou 7, comprenant au moins un dispositif d'alarme pour émettre une alarme, le dispositif d'alarme étant prévu pour émettre une alarme dans le cas où le résultat de l'exécution du procédé selon l'une quelconque des revendications 1 à 5 ne se situe pas dans une plage de valeurs ou dans une plage prédéterminée(s) .

9. Un appareil de traitement médical qui comprend au moins un dispositif de détection selon la revendication 6 et/ou qui est en transmission de signal ou est relié pour transmission de signal avec celui-ci.

10. L'appareil de traitement médical selon la revendication 9, qui est conçu sous la forme d'un appareil de traitement du sang, notamment comme un appareil d'aphérèse ou de dialyse, notamment comme un appareil d'hémodialyse, d'hémofiltration, d'hémodiafiltration.

11. L'appareil de traitement médical selon l'une quelconque des revendications 9 et 10, où au moins un des corps de déplacement (25, 27) est conçu comme un galet, et où la pompe tubulaire (23) est conçue comme une pompe à galets.

12. Un support de stockage numérique, notamment sous la forme d'un disque, d'un CD ou d'un DVD ou d'une EPROM, avec des signaux de commande lisibles électroniquement, configuré pour interagir avec l'appareil de traitement selon la revendication 9, de manière à déclencher les étapes du procédé fondé sur l'invention selon l'une quelconque des revendications 1 à 5.

13. Un produit de programme informatique comprenant un code de programme enregistré sur un support lisible par machine pour déclencher les étapes du procédé fondé sur l'invention selon l'une quelconque des revendications 1 à 5 lorsque le produit de programme informatique s'exécute sur l'appareil de traitement selon la revendication 9.

14. Un programme informatique comprenant un code de programme pour déclencher les étapes automatiques d'un procédé fondé sur l'invention selon l'une quelconque des revendications 1 à 5 lorsque le programme informatique s'exécute sur l'appareil de traitement selon la revendication 9.
